(19) Europäisches Patentamt
European Patent Office
Office européen des brevets

(11) Publication number: **0 019 866**
**B1**

(12) **EUROPEAN PATENT SPECIFICATION**

(45) Date of publication of patent specification: **14.03.84**

(51) Int. Cl.$^3$: **C 07 D 451/02**
//C07C119/14, C07C143/79

(21) Application number: **80102868.9**

(22) Date of filing: **23.05.80**

(54) **Process for the preparation of 10,11-dihydro-5H-dibenzo (a,d)cyclohepten-5,10-imines.**

(30) Priority: **04.06.79 US 45494**

(43) Date of publication of application:
**10.12.80 Bulletin 80/25**

(45) Publication of the grant of the patent:
**14.03.84 Bulletin 84/11**

(84) Designated Contracting States:
**AT LU NL**

(56) References cited:
BE - A - 870 562
US - A - 3 892 756

JOURNAL OF HETEROCYCLIC CHEMISTRY, vol. 13, no. 6, December 1976 Provo Utah US J.H. DYGOS: "The synthesis of syn- and anti- 11-hydroxy-12-methyl-10,11-dihydro-5,10-imino (5H)dibenzo(a,d)cycloheptene, pages 1355-57.

(73) Proprietor: **MERCK & CO. INC.**
**126, East Lincoln Avenue P.O. Box 2000**
**Rahway New Jersey 07065 (US)**

(72) Inventor: **Shepard, Kenneth L.**
**726 Marietta Drive**
**Ambler, Pa 19002 (US)**
Inventor: **Brenner, Daniel G.**
**11 Pitcher Avenue**
**Medford, MA 02155 (US)**

(74) Representative: **Blum, Rudolf Emil Ernst et al,**
**c/o E. Blum & Co Patentanwälte Vorderberg 11**
**CH-8044 Zürich (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 019 866 B1

Process for the preparation of 10,11-dihydro-5H-dibenzo[a,d]cyclohepten-5,10-imines

## BACKGROUND OF THE INVENTION

This invention is concerned with a novel process for the synthesis of 10,11-dihydro-5H-dibenzo[a,d]cyclohepten-5,10-imines which are useful as antianxiety agents, muscle relaxants, and in the treatment of extrapyramidal disorders such as in Parkinson's disease. The products of the novel process have general structural formula:

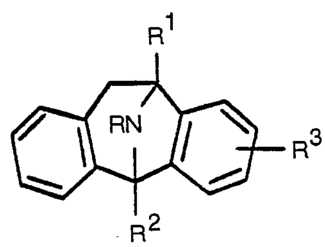

Structurally related compounds are known in the art to have qualitatively similar utilities. For example U.S. 3,892,756 discloses 10,11-dihydro-5H-dibenzo[a,d]cyclohepten-5,10-imine and derivatives, and U.S. Patent 4,064,139 discloses 9,10-dihydro-anthracen-9,10-imines and derivatives. In addition many of the compounds preferable by the novel process of this invention are disclosed in Belgian patent 870 562. In examples 1 and 8 of said Belgian patent the 10-amino compound, is cyclized by addition across the double bond of the exocyclic 5-methylene group according to the following reaction scheme:

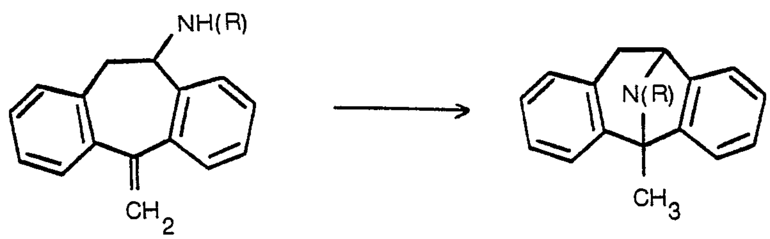

According to example 1 R is a hydroxy group and according to example 8 R is the methyl group and in this latter case the cyclization is performed using an organometallic compound, i.e. n-butyl-lithium. The process described in said Belgian patent, however, requires many reaction steps. The starting material used in example 1 of said Belgian patent is the 10-bromo-5H-dibenzo[a,d]cyclohepten-5-one which has to be prepared from the 5H-dibenzo[a,d]cyclohepten-5-one according to the method of Treibs et al., Chem. Ber., 84, 671—679 (1951) in two steps starting from 5H-dibenzo[a,d]cyclohepten-5-on according to the following reaction scheme:

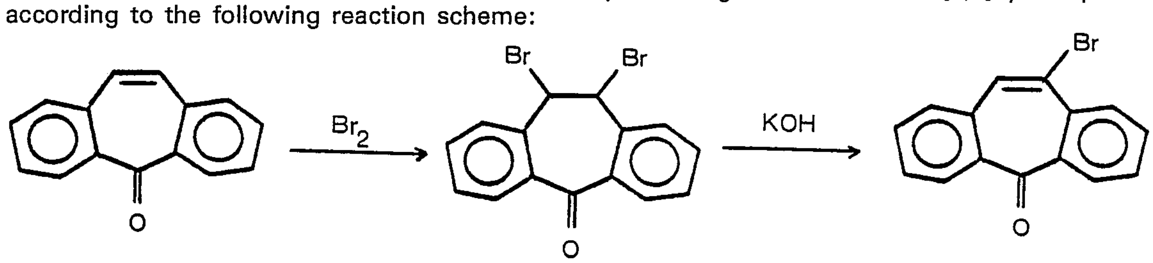


and the so resulting 10-bromo compound, thereafter, has to be submitted to the 7 steps A—G described in example 1 of the Belgian patent in order to obtain the desired 10,11-dihydro-5H-dibenzo[a,d]cyclohepten-5,10-imine.

## Detailed description of the invention

The object of the present invention is a novel process for the preparation of 10,11-dihydro-5H-dibenzo[a,d]cyclohepten-5,10-imines which requires far less reaction steps than the prior art processes for the preparation of such compounds. According to the inventive process a compound of structural formula I

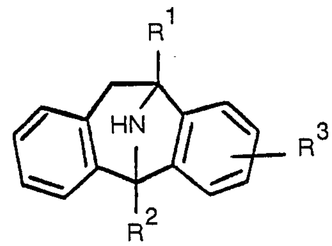

wherein

$R^1$ is hydrogen, or

$R^1$ and $R^2$ are independently

(1) $C_{1-5}$ alkyl,

(2) $C_{2-5}$ alkenyl,

(3) phenyl-$C_{1-3}$ alkyl,

(4) $C_{3-6}$ cycloalkyl, or

(5) $C_{3-6}$ cycloalkyl-$C_{1-3}$ alkyl; and

$R^3$ is

(1) hydrogen, or

(2) halogen;

or a pharmaceutically acceptable salt of the compound of structural formula I is prepared.

The new inventive process comprises the following steps in sequence:

(1) the treatment of an acylamide having the formula II

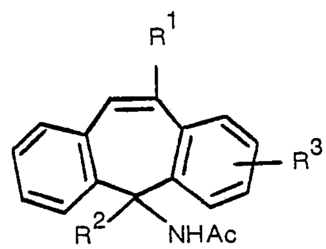

II

with acid or base to produce the acylimine of formula III

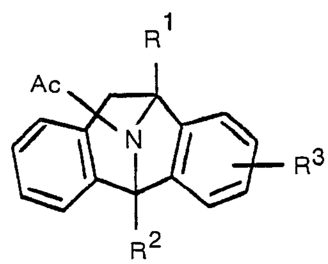

III

and

(2) followed by the treatment of the acylimine of formula III with a strong acid or strong base, or where Ac is an arylsulfonyl group with a strong acid or a reducing agent, to produce the imine of formula I or a pharmaceutically acceptable salt thereof.

A preferred compound prepared according to the inventive process is a compound of formula I wherein $R^1$ is hydrogen, $R^2$ is methyl and $R^3$ is hydrogen.

In the used starting materials of formula II Ac is any desired acyl group including arylsulfonyl groups, and a preferred acyl group is the tosyl group.

In step (1) of the inventive process the imine bridge is formed by the addition of the acylamine group across the conjugated double bond. This cyclization is preferably performed by a treatment with a base. Preferably this formation of the imine bridge is effected by a treatment with an alkali metal hydroxide, specially sodium or potassium hydroxide in a high boiling ethereal type solvent such as diglyme. Preferably reaction temperatures and times of about 150 to about 170°C for about 24 to about 48 hours are employed.

In the reaction step (2) of the inventive process, the acyl group of the compounds of formula II is removed. Said deacylation of the imine group is performed by acid or base hydrolysis, using a strong acid or a strong base. Preferably said treatment is performed at a temperature of 25°C to about 150°C for 2 to 48 hours, preferably 6 to 24 hours. Strong mineral acids are preferred, specially hydrochloric acid or sulfuric acid or a mixture of a strong mineral acid with an organic acid, preferably acetic acid.

If in step (2) of the inventive process, the acyl group to be removed is an arylsulfonyl group, then this removal is performed with a strong acid or reducing agent, preferably using a reducing agent performing a hydrogenolysis. This hydrogenolysis can be performed with an excess of sodium bis(2-methoxyethoxy)aluminum hydride in an inert organic solvent such as toluene at about 15°C to about 50°C, preferably at 20°C to about 30°C for 6—48 hours, preferably about 12—24 hours.

According to a preferred embodiment of the inventive process, the starting material of formula II

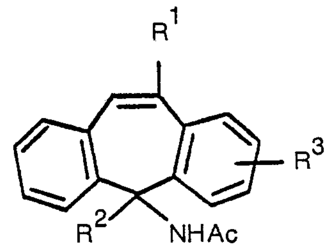

II

is produced performing the following steps in sequence:

(a) the treatment of a compound of formula IV

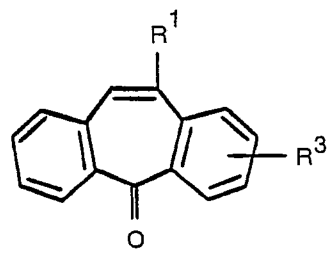

IV

with gaseous ammonia in the presence of titanium tetrachloride, resulting in an imine of formula V

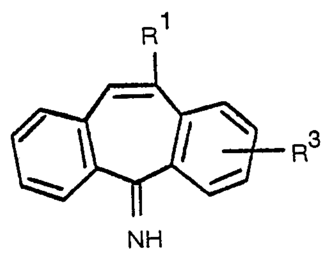

V

(b) the treatment of the imine of formula V with an acylating agent, preferably in the presence of an acid acceptor, resulting in an acylimine of formula VI

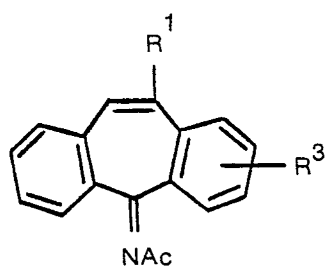

VI

and

(c) the treatment of the acylimine of formula VI with an organolithium compound of formula VII

$R^2$ Li VII

introducing the radical $R^2$ and forming the acylamide starting material of formula II which is thereafter

submitted to steps 1 and 2 of the above mentioned two-step process. Said reaction is illustrated by the following reaction scheme A.

REACTION SCHEME A

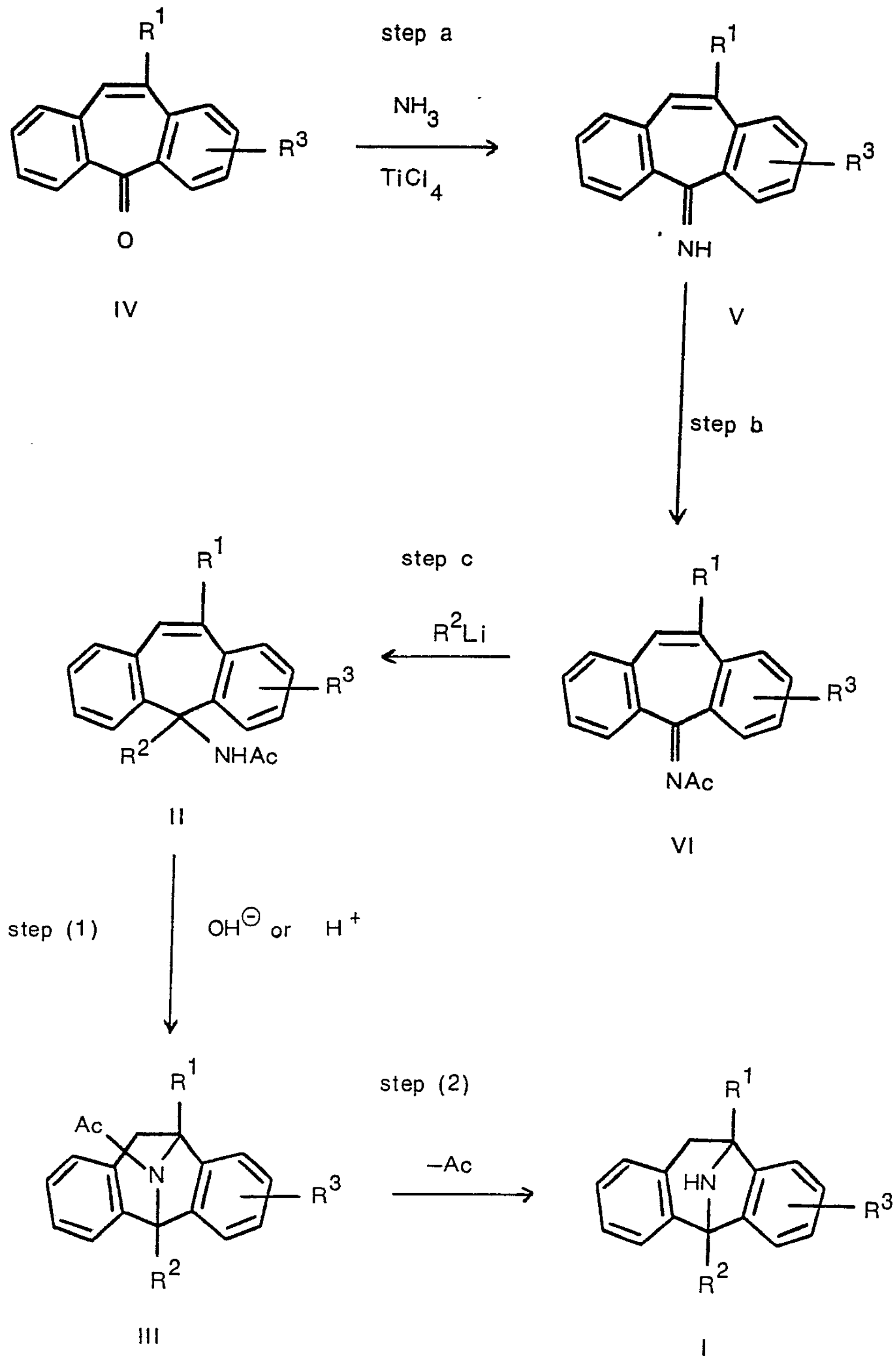


IV

V

VI

II

III

I

If in said reaction scheme A in the starting material of formula IV $R^1$ and $R^3$ are hydrogen atoms, then said starting material is a 5H-dibenzo[a,d]cyclohepten-5-one and, accordingly, identical with the ketone used for the preparation of the 10-bromo-5H-dibenzo[a,d]cyclohepten-5-one which is the starting material of example 1 according to the process of the Belgian patent 870,562. The inventive process according to the above reaction scheme A, accordingly, requires only totally 5 reaction steps in

order to produce the desired final material of formula I compared with the process of the corresponding Belgian patent which requires totally 9 reaction steps. The inventive process, accordingly, represents an important improvement over the process of the Belgian patent.

In step (a) of the process of the above reaction scheme A for the preparation of the starting material of formula II, the ketone of formula IV is treated with gaseous ammonia in the presence of titanium tetrachloride forming the unexpectedly stable imine of formula V. Said reaction is preferably conducted in an inert organic solvent in which the starting materials are soluble, specially an aromatic solvent such as toluene or benzene. The temperature is not critical and may be at about −10°C to about +50°C preferably between ice-bath and room temperatures. Generally, times from 2 to about 10 hours are required usually 3 to about 5 hours.

In step (b) of said process, the free imine of formula V is acylated by a treatment with an acylating agent yielding the resulting acyl imine of formula VI. As acylating agent there can be used an acyl halide such as tosyl chloride, benzene-sulfonyl chloride, $C_{1-3}$ lower alkanoyl chloride, benzoyl chloride, or a $C_{1-3}$ lower alkyl chloroformate. Generally, standard acylating conditions are employed such as contacting the two reagents in an inert organic solvent in the presence of an acid acceptor such as an organic base, specially pyridine which can also be used as the solvent, triethylamine, or an inorganic base, specially an alkali metal carbonate, or an alkaline resin. Preferably reaction times and temperatures of 1 to 6 hours at about 0°C to about 50°C, specially 2 to 4 hours at ice bath temperature to room temperature are employed, although prolonged reaction times are not detrimental.

Step (c) of the process for the preparation of the starting material of formula II comprises the addition of $R^2H$ across the imine double bond. The acyl imine of formula VI is treated with the organolithium compound of formula VI introducing the radical $R^2$ and forming the acyl amide starting material of formula II. The treatment of the acyl imine of formula VI with the organolithium compound of formula VII is preferably performed in an inert organic solvent, specially an ether such as diethyl ether, tetrahydrofuran or 1,2-dimethoxyethane. The reaction temperature is preferably 0°C to about 50°C, specially room temperature and the reaction time generally 0.5—4 hours, preferably about 1—3 hours. Prolonged stirring times are not detrimental.

The compounds preparable by the novel process of this invention are capable of producing anxiety relief without causing excessive sedation or sleep at dosage level of from about 0.01 to about 50 mg per kilogram of body weight preferably about 0.5—10 mg/kg of body weight on a regimen of 1—4 times a day. In addition, they are useful as muscle relaxants, anticonvulsants and in the treatment of extrapyramidal disorders when indicated at comparable dosage levels. The exact treatment level will depend upon the case history of the animal or human individual being treated and in the last analysis the precise treatment level falling within the above guidelines is left to the discretion of the therapist.

Pharmaceutical compositions comprising the imines prepared by the novel process of this invention are preferably in unit dosage forms such as tablets, pills, capsules, powders, granules, sterile parenteral solutions or suspensions, or suppositories for oral, parenteral or rectal administration.

## Example 1

5-Methyl-10,11-Dihydro-5H-Dibenzol[a,d,]cyclohepten-5,10-Imine

Step A: Preparation of 5H-dibenzo[a,d]cyclohepten-5-imine

Ammonia gas is bubbled slowly over a thirty minute period into an ice-cooled, stirred mixture of 25 g of 5H-dibenzo[a,d]cyclohepten-5-one and 10 ml of titanium tetrachloride in 750 ml of toluene. The cooling bath is removed and stirring is continued while the mixture warms to room temperature. The mixture is then refluxed 5½ hours. The mixture is added to one liter of saturated aqueous sodium carbonate solution with stirring. The toluene layer is washed with 500 ml of saturated sodium carbonate solution and 300 ml of saturated sodium chloride solution. The original aqueous layer is extracted with 2 x 500 ml of ethylacetate and the combined extracts are washed with saturated sodium carbonate and sodium chloride. The toluene phase and ethyl acetate extracts are combined and dried over anhydrous sodium sulfate and concentrated to dryness. The residue on trituration with petroleum ether gave 20.6 g of 5H-dibenzo[a,d]cyclohepten-5-imine, m.p. 60—62°C.

Step B: Preparation of 5-*p*-toluenesulfonimino-5H-dibenzo[a,d]cycloheptene

*p*-Toluenesulfonyl chloride (22.3 g) is added portionwise to an ice-cooled, stirred solution of 24 g of 5H-dibenzo[a,d]cyclohepten-5-imine in 300 ml of pyridine. The ice-bath is removed and stirring is continued at room temperature (~20°C) over the weekend. Methylene chloride (600 ml) is added and the mixture is extracted with 3 x 500 ml of 1N hydrochloric acid. The methylene chloride phase is dried over anhydrous sodium sulfate and concentrated to dryness. The residue (39.2 g) is recrystallized by dissolving in 800 ml of hot ethanol, filtering, concentrating to 500 ml and cooling to give 16.7 g 5-*p*-toluenesulfonimino-5H-dibenzo[a,d]cycloheptene, which on recrystallization from ethanol has m.p. 156.5—158.5°C.

Step C: Preparation of 5-methyl-5-*p*-toluenesulfonamido-5H-dibenzo[a,d]cycloheptene

A solution of 5.0 g of 5-*p*-toluenesulfonimino-5H-dibenzo[a,d]cycloheptene in 75 ml of dry

tetrahydrofuran is stirred under nitrogen and treated dropwise with 30 ml of 1.4M methyllithium in ether. After stirring overnight at room temperature under nitrogen, the reaction mixture is added to 100 ml of 10% (w/v) aqueous ammonium chloride. The mixture is extracted with ethyl acetate. The extract is washed with water and saturated sodium chloride solution, filtered, dried over anhydrous sodium sulfate and concentrated to dryness to give 6.4 g product which after recrystallization from ethanol gives 2.8 g of 5-methyl-5-*p*-toluenesulfonamido-5H-dibenzo[a,d]cycloheptene, m.p. 175—178°C.

Step D: Preparation of 5-methyl-12-*p*-toluenesulfonyl-5H-dibenzo[a,d]cyclohepten-5,10-imine

A mixture of 1 g of 5-methyl-5-*p*-toluenesulfonamido-5H-dibenzo[a,d]cycloheptene and 5 g of potassium hydroxide pellets (86.1%) in 50 ml of diglyme is heated in an oil bath at 160—170°C for 42 hours. The mixture is added to 200 ml of water and neutralized (pH 7) with concentrated hydrochloric acid. The solution is extracted with 2 x 200 ml of ether and each of the ether extracts are back washed with 3 x 100 ml of water. The ether extracts are separately dried over anhydrous sodium sulfate, filtered and concentrated to dryness to give 330 mg and 100 mg respectively of product. Recrystallization from ethanol and drying provides 5-methyl-12-*p*-toluenesulfonyl-5H-dibenzo[a,d]cyclohepten-5,10-imine, m.p. 186.5—188.5°C.

Step E: Preparation of 5-methyl-10,11-dihydro-5H-dibenzo[a,d]cyclohepten-5,10-imine

A mixture of 290 mg of 5-methyl-12-*p*-toluenesulfonyl-5H-dibenzo[a,d]cyclohepten-5,10-imine, 5 ml of concentrated hydrochloric aid, and 5 ml of glacial acetic acid is refluxed overnight. The clear solution is added to 100 ml of water and 20% (w/v) aqueous sodium hydroxide is added to pH 9. The mixture is extracted with 3 x 100 ml of methylene chloride. The extract is washed with saturated aqueous sodium chloride solution, dried over anhydrous sodium sulfate and evaporated to give 210 mg of oil. The oil is dissolved in acetone and treated with 142 mg of oxalic acid in acetone. The mixture is cooled and the precipitate is collected to give 100 mg 5-methyl-10,11-dihydro-5H-dibenzo[a,d]cyclohepten-5,10-imine, m.p. 211—212°C.

Following the procedure substantially as described in Example 1, but substituting for the 5H-dibenzo[a,d]cyclohepten-5-one used in Step A, and the methyllithium used in Step C, the $R^1$—$R^3$—5H-dibenzo[a,d]cyclohepten-5-ones and the organolithiums of formula $R^2Li$ described in Table I there are produced the $R^1$—$R^2$—$R^3$-10,11-dihydro-5H-dibenzo[a,d]cyclohepten-5,10-imine also described in Table I in accordance with the processes of Reaction Scheme A.

TABLE I

| $R^1$ | $R^3$ | $R^2$ |
|---|---|---|
| $CH_3-$ | 3-Br | $CH_3-$ |
| $n-C_3H_7$ | 2-Br | $n-C_3H_7-$ |
| $CH_2=CH-CH_2-$ | H | 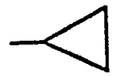 |
| 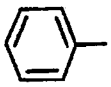 $-CH_2-$ | H | $C_2H_5-$ |
| 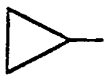 | 6-Br | $CH_2=CHCH_2-$ |
| 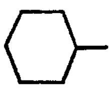 | H | 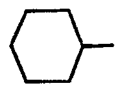 $-CH_2-$ |
| 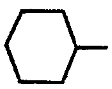 $-CH_2-$ | 7-Br | 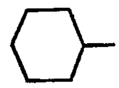 |
| 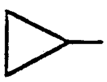 $-CH_2-$ | H | $CH_3-$ |
| $C_2H_5-$ | 2-F | $CH_3-$ |
| $CH_3-$ | 3-F | 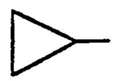 $-CH_2-$ |
| H | 3-Br | $CH_3-$ |
| H | 7-Br | $CH_3-$ |
| H | 3-F | $CH_3-$ |

Example 2

Tablet Preparation

Tablets containing 1.0, 2.0, 25.0, 26.0, 50.0, and 100.0 mg, respectively, of 10,11-dihydro-5-methyl-5H-dibenzo[a,d]cyclohepten-5,10-imine are prepared as illustrated below.

TABLE FOR DOSES CONTAINING FROM 1—25 MG OF THE ACTIVE COMPOUND

| | Amount — mg | | |
|---|---|---|---|
| 10,11-dihydro-5-methyl-5H-dibenzo[a,d]cyclohepten-5,10-imine | 1.0 | 2.0 | 25.0 |
| Microcrystalline cellulose | 49.25 | 48.75 | 37.25 |
| Modified food corn | 49.25 | 48.75 | 37.25 |
| Magnesium stearate | 0.50 | 0.50 | 0.50 |

TABLE FOR DOSES CONTAINING FROM 26—100 MG OF THE ACTIVE COMPOUND

| | Amount — mg | | |
|---|---|---|---|
| 10,11-dihydro-5-methyl-5H-dibenzo[a,d]cyclo-hepten-5,10-imine | 26.0 | 50.0 | 100.0 |
| Microcrystalline cellulose | 25.0 | 100.0 | 200.0 |
| Modified food corn starch | 2.21 | 4.25 | 8.5 |
| Magnesium stearate | .39 | 0.75 | 1.5 |

All of the active compound, lactose, and a portion of the corn starch are mixed and granulated to a 10% corn starch paste. The resulting granulation is sieved, dried and blended with the remainder of the corn starch and the magnesium stearate. The resulting granulation is then compressed into tablets containing 1.0 mg, 2.0 mg, 25.0 mg, 26.0 mg, 50.0 mg, and 100.0 mg of active ingredient per tablet.

**Claims**

1. A process for the preparation of a compound of structural formula I

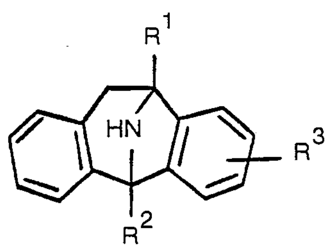

I

wherein
$R^1$ is hydrogen or
$R^1$ and $R^2$ are independently
(1) $C_{1-5}$ alkyl,
(2) $C_{2-5}$ alkenyl,
(3) phenyl-$C_{1-3}$ alkyl,
(4) $C_{3-6}$ cycloalkyl, or
(5) $C_{3-6}$ cycloalkyl-$C_{1-3}$ alkyl; and
$R^3$ is
(1) hydogen, or
(2) halogen;

and pharmaceutically acceptable salts of the compounds of formula I which comprises the following steps in sequence:

(1) the treatment of an acylamide having the formula II

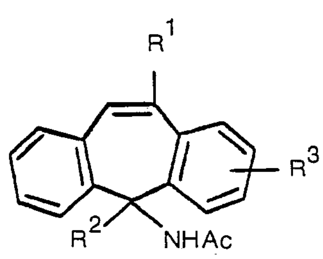

II

with acid or base to produce the acylamine of formula III

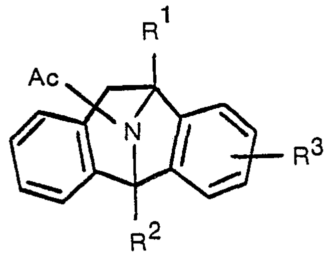

III

and

(2) followed by the treatment of the acylimine of formula III with a strong acid or strong base, or where Ac is an arylsulfonyl group with a strong acid or a reducing agent, to produce the imine of formula I or a pharmaceutically acceptable salt thereof.

2. Process of claim 1 for the preparation of a compound of formula I wherein $R_1$ is hydrogen, $R^2$ is methyl and $R^3$ is hydrogen.

3. Process of claim 1 or 2 wherein a starting material of formula II is used in which Ac is tosyl.

4. Process of one of the claims 1—3 wherein in step (1) the cyclization is performed with an alkali metal hydroxide.

5. Process according to patent claim 4 wherein in step (1) the cyclization is performed with the alkali metal hydroxide in a high boiling ethereal type solvent.

6. Process according to one of the claims 1—5 wherein in step (2) the acyl group is removed using a strong mineral acid or a mixture of a strong mineral acid with an organic acid.

7. Process according to claim 6 wherein as strong mineral acid there is used hydrochloric acid or sulphuric acid and if mixtures with an organic acid are used, said organic acid is acetic acid.

8. Process according to one of the claims 1—7 wherein an acyl group being an arylsulfonyl group is removed using a reducing agent performing a hydrogenolysis.

9. A process according to claim 8 wherein the arylsulfonyl group is removed with an excess of sodium bis(2-methoxyethoxy)aluminum hydride in an inert organic solvent.

10. Process according to one of the claims 1—9 wherein the starting material of formula II

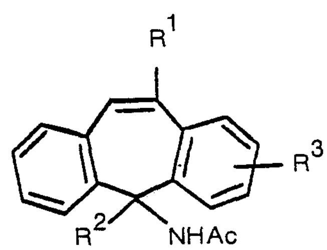

II

is produced performing the following steps in sequence:

(a) the treatment of a compound of formula IV

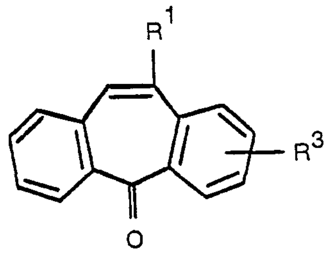

IV

with gaseous ammonia in the presence of titanium tetrachloride, resulting in an imine of formula V

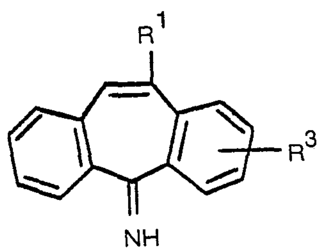

V

(b) the treatment of the imine of formula V with an acylating agent resulting in an acylimine of formula IV

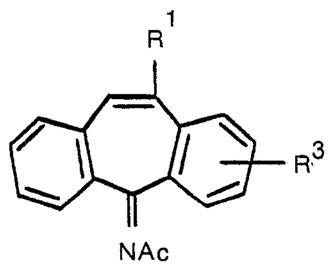

VI

and

(c) the treatment of the acylimine of formula VI with an organolithium compound of formula VII

$R^2Li$ VII

introducing the radical $R^2$ and forming the acylamide starting material of formula II which is thereafter submitted to steps 1 and 2 of the process of claim 1.

**Patentansprüche**

1. Ein Verfahren zur Herstellung einer Verbindung der Strukturformel I

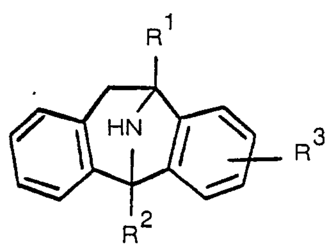

I

worin

$R^1$ Wasserstoff ist oder

$R^1$ und $R^2$ unabhängig voneinander

(1) $C_{1-5}$-Alkyl,

(2) $C_{2-5}$-Alkenyl,

(3) Phenyl-$C_{1-3}$-alkyl,

(4) $C_{3-6}$-Cycloalkyl oder

(5) $C_{3-6}$-Cycloalkyl-$C_{1-3}$-alkyl sind; und

$R^3$

(1) Wasserstoff oder

(2) Halogen ist;

und pharmazeutische annehmbarer Salze der Verbindungen der Formel I, umfassend die Folge der folgenden Stufen:

(1) die Behandlung eines Acylamids der Formel II

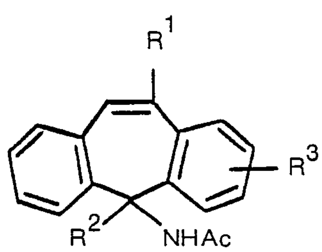

II

mit einer Säure oder Base zur Bildung des Acylimins der Formel III

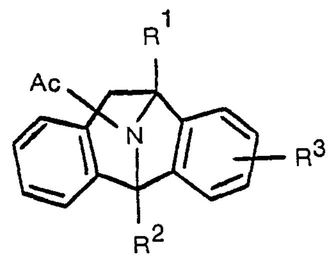

III

und

(2) gefolgt von der Behandlung des Acylimins der Formel III mit einer starken Säure oder starken Base, oder, falls Ac eine Arylsulfonylgruppe ist, mit einer starken Säure oder einem Reduktionsmittel, zur Bildung des Imins der Formel I oder eines pharmazeutisch annehmbaren Salzes davon.

2. Verfahren nach Anspruch 1 zur Herstellung einer Verbindung der Formel I, worin $R^1$ Wasserstoff ist, $R^2$ Methyl ist und $R^3$ Wasserstoff ist.

3. Verfharen nach Anspruch 1 oder 2, worin ein Ausgangsmaterial der Formel II verwendet wird, in welcher Ac Tosyl ist.

4. Verfahren nach einem der Ansprüche 1 bis 3, worin in Stufe (1) die Cyclisierung mit einem Alkalimetallhydroxid bewirkt wird.

5. Verfahren nach Patentanspruch 4, worin in Stufe (1) die Cyclisierung mit dem Alkalimetallhydroxid in einem hochsiedenden Lösungsmittel vom Ethertypus ausgeführt wird.

6. Verfahren nach einem der Ansprüche 1 bis 5, worin in Stufe (2) die Acylgruppe unter Anwendung einer starken Mineralsäure oder eines Gemisches einer starken Mineralsäure mit einer organischen Säure entfernt wird.

7. Verfahren nach Anspruch 6, worin als starke Mineralsäure Chlorwasserstoffsäure oder Schwefelsäure angewendet wird, und, falls Gemische mit einer organischen Säure angewendet werden, die genannte organische Säure Essigsäure ist.

8. Verfahren nach einem der Ansprüche 1 bis 7, worin eine Acylgruppe, die eine Arylsulfonylgruppe ist, unter Anwendung eines eine Hydrogenolyse bewirkenden Reduktionsmittels entfernt wird.

9. Verfahren nach Anspruch 8, worin die Arylsulfonylgruppe mit einem Überschuß von Natrium-bis(2-methoxyethoxy)aluminiumhydrid in einem inerten organischen Lösungsmittel entfernt wird.

10. Verfahren nach einem der Ansprüche 1 bis 9, worin das Ausgangsmaterial der Formel II

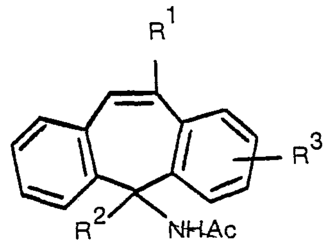

II

durch aufeinanderfolgende Ausführung der folgenden Stufen gebildet wird:

(a) die Behandlung einer Verbindung der Formel IV

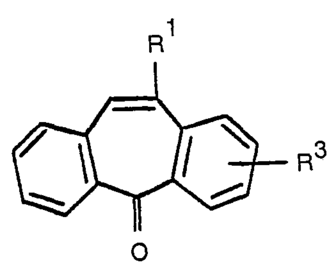

IV

mit gasförmigem Ammoniak in Gegenwart von Titantetrachlorid, wobei ein Imin der Formel V

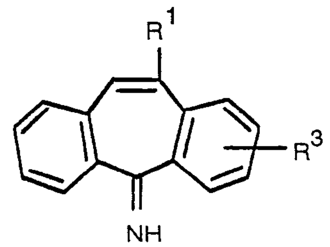

V

entsteht,

(b) die Behandlung des Imins der Formel V mit einem Acylierungsmittel, wobei ein Acylimin der Formel VI

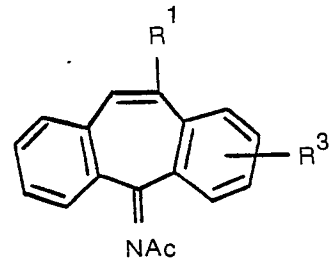

VI

entsteht, und

(c) die Behandlung des Acylimins der Formel VI mit einer Organolithiumverbindung der Formel VII

$R^2Li$ VII

unter Einführung des Restes $R^2$ und Bildung des Acylamidausgangsmaterials der Formel II, welches dann den Stufen 1 und 2 des Verfahrens des Anspruchs 1 unterworfen wird.

**Revendications**

1. Procédé pour la préparation d'un composé de formule I

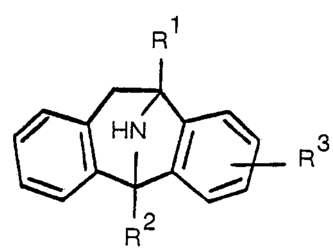

I

dans laquelle:

$R^1$ représente de l'hydrogène, ou

$R^1$ et $R^2$ sont indépendamment

(1) alkyle en $C_1$ à $C_5$,

(2) alkényle en $C_2$ à $C_5$,

(3) phényl-(alkyle en $C_1$ à $C_3$),

(4) cycloalkyle en $C_3$ à $C_6$, ou

(5) (cycloalkyle en $C_3$ à $C_6$)-(alkyle en $C_1$ à $C_3$), et

$R^3$ représente

(1) de l'hydrogène ou

(2) un halogène,

et des sels pharmaceutiquement acceptables des composés de formule I, caractérisé en ce qu'il comprend les étapes séquentielles suivantes:

(1) traitement d'un acylamide de formule II

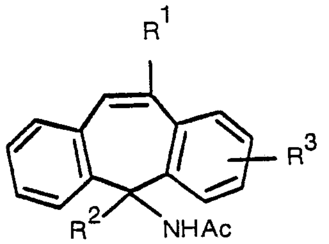

II

avec un acide ou une base, pour produire l'acylimine de formule III

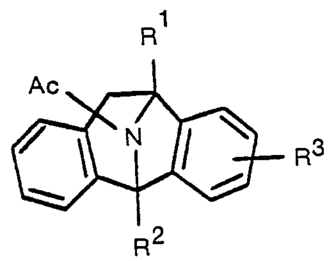

III

et (2) suivi du traitement de l'acylimine de formule III avec un acide ou une base forts, ou, lorsque Ac est un groupement arylsulfonyle, avec un acide fort ou un agent réducteur, pour produire l'imine de formule I ou d'un sel pharmaceutiquement acceptable de cet imine.

2. Procédé selon la revendication 1, pour la préparation d'un composé de formule I dans laquelle $R^1$ est de l'hydrogène, $R^2$ est méthyle, et $R^3$ est de l'hydrogène.

3. Procédé selon la revendication 1 ou 2, dans lequel on utilise un produit de départ de formule II dans laquelle Ac est un groupement tosyle.

4. Procédé selon l'un des revendications 1 à 3 dans lequel la cyclisation de l'étape (1) est exécutée au moyen d'un hydroxyde de métal alcalin.

5. Procédé selon la revendication 4 dans lequel la cyclisation de l'étape (1) est executée au moyen de l'hydroxyde de métal alcalin au sein d'un solvant éthéré à haut point d'ébullition.

6. Procédé selon l'une des revendications 1 à 5, dans lequel le groupement acyle est enlevé dans l'étape (2) au moyen d'un acide minéral fort ou d'un mélange d'acide minéral fort avec un acide organique.

7. Procédé selon la revendication 6, dans lequel on utilise, comme acide minéral fort, de l'acide chlorhydrique ou sulfurique, et, si l'on utilise des mélanges avec un acide organique, celui-ci est l'acide acétique.

8. Procédé selon l'une des revendications 1 à 7, dans lequel un groupement acyle qui est un groupement arylsulfonyle est enlevé au moyen d'un agent réducteur opérant une hydrogénolyse.

9. Procédé selon la revendication 8, dans lequel le groupement arylsulfonyle est enlevé au moyen d'un excès d'hydrure de bis-(2-méthoxyéthoxy)-aluminium sodique au sein d'un solvant organique inerte.

10. Procédé selon l'une des revendications 1 à 9, dans lequel le produit de départ de formule II

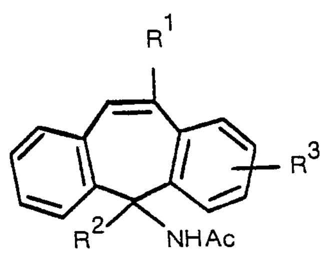

II

est obtenu par les étapes séquentielles suivantes:

(a) traitement d'un composé de formule IV

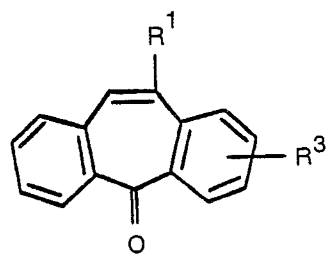

IV

avec de l'ammoniac gazeux en présence de tétrachlorure de titanium, donnant une imine de formule V

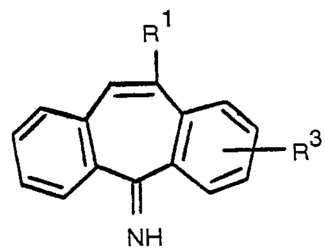

V

(b) traitement de l'imine de formule V avec un agent d'acylation, donnant un acylimine de formule VI

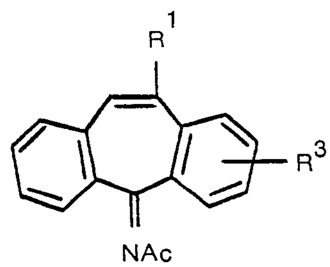

VI

(c) traitement de l'acylamine de formule VI avec un composé organolithié de formule VII

$R^2Li$ (VII)

pour introduire le groupement $R^2$ et former l'acylamide de départ, de formule II, qui est alors soumis aux étapes 1 et 2 du procédé selon la revendication 1.